# EUROPEAN PATENT APPLICATION

(11) **EP 0 194 048 A1**
(43) Date of publication of application: **10.09.1986**
(21) Application number: 86300888.4
(22) Date of filing: 10.02.1986
(51) Int. Cl.: C07D 211/90, C07D 401/12, C07D 413/04, A61K 31/44

(54) **Dihydropyridine alkanol amines, process for their preparation and pharmaceutical compositions containing them**

(30) Priority: 11.02.1985 GB 8503429
(71) Applicant: IMPERIAL CHEMICAL INDUSTRIES PLC, London SW1P 3JF (GB)
(72) Inventor: Hargreaves, Rodney Brian, Cheshire (GB); McLoughlin, Bernard Joseph, Cheshire (GB); Mills, Stuart Dennett, Macclesfield, S11 9RD (GB); Taylor, Melvin Joseph, Cheshire (GB)
(74) Representative: Slatcher, Reginald Peter (GB)

(57) **Abstract**

A dihydropyridine of the formula: 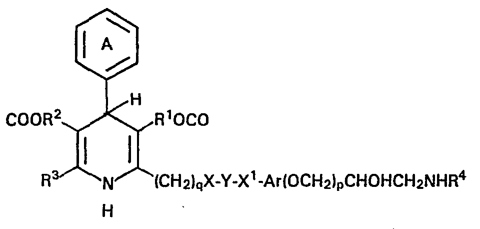 wherein R¹ and R² each is alkyl or alkoxyalkyl, wherein R³ is alkyl, wherein R⁴ is alpha-branched-chain alkyl, hydroxyalkyl, arylalkyl, aryloxyalkyl or acylaminoalkyl, wherein benzene ring A bears one or more substituents selected from halogeno, cyano, nitro, trifluoromethyl and alkyl or bears the substituent =N-O-N= attached to the 2- and 3-positions; wherein Ar is phenylene, naphthylene, tetrahydronaphthylene, indanylene or pyridylene which is unsubstituted or which bears one or more substituents, wherein p is 0 or 1;
wherein q is 1, 2, 3 or 4;
wherein X is -O-, -NH-, -NHCO- or -CONH;
wherein X¹ is a direct link or is -O-, -S-, -NH- or -NHS0₂-; and wherein Y is straight- or branched-chain alkylene which may optionally be interrupted by one or two groups selected from oxygen, sulphur, imino, substituted imino, phenylene, substituted phenylene, pyridylene, cycloalkylene, 1,4-piperazinediyl, 1,4-piperidinediyl and amido groups; or an acid-addition salt thereof, process for their manufacture and pharmaceutical composition containing them. The compound possess either beta-adrenergic blocking or calcium ion slow channel blocking properties, or both such properties, and may be used in the treatment of hypertension.

## Description

This invention relates to new chemical compounds and more particularly it relates to new dihydropyridine derivatives which possess antihypertensive properties.

Many 2,6-dialkyl-4-aryl-1,4-dihydropyridine-3,5-dicarboxylate derivatives are known which inhibit the movement of calcium ions in the cardiovascular system of warm-blooded animals, and which thereby produce an antihypertensive effect. The most-used of these is nifedipine, which is dimethyl 1,4-dihydro-2,6-dimethyl-4-o-nitrophenylpyridine-3,5-dicarboxylate.

Also known are many 1-aryloxy-3-amino-propan-2-ol derivatives which possess beta-adrenergic receptor blocking properties and which also produce an antihypertensive effect. Two of the most-used of these are propranolol and atenolol, which are respectively 1-(naphth-1-yloxy)- and I-p-carbamoylmethylphenoxy-3-isopropylaminopropan-2-ol.

The only described attempt to combine these two types of chemical structure into one molecule is reported by Merck workers in the Journal of Medicinal Chemistry, 1981, Vol. 24, pages 628 to 631, in which a 3-amino-2-hydroxypropoxy substituent was introduced into the 4-aryl substituent of a 4-aryl-l,4-dihydropyridine derivative, without much success in producing a compound with antihypertensive activity of the type sought by the authors.

We have now found that compounds which do possess useful antihypertensive activity may be obtained by suitably combining a 3-aryloxy-2-hydroxypropylamino moiety with a 1,4-dihydropyridine moiety.

According to the present invention there is provided a dihydropyridine of the formula: 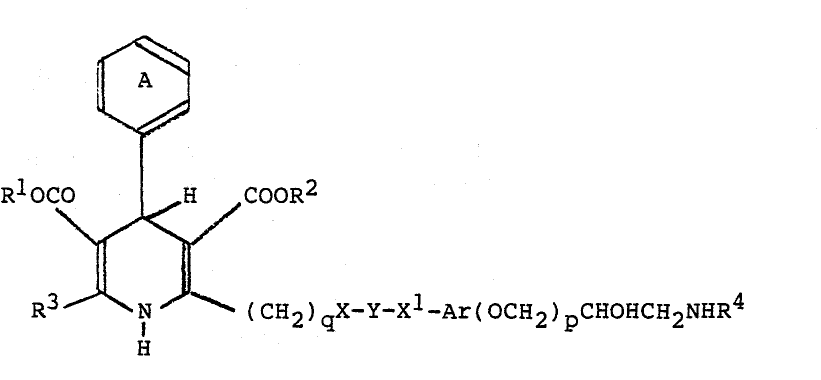
wherein R¹ and R², which may be the same or different, each is alkyl or alkoxyalkyl each of up to 6 carbon atoms:
wherein R³ is alkyl of up to 6 carbon atoms;
wherein R⁴ is alpha-branched-chain alkyl or hydroxyalkyl each of up to 6 carbon atoms, or alpha-branched chain arylalkyl or aryloxyalkyl each of up to 12 carbon atoms, or acylaminoalkyl wherein the acyl is is of up to 10 carbon atoms and the alkyl is of up to 6 carbon atoms; wherein benzene ring A bears one or more substituents selected from halogeno, cyano, nitro, trifluoromethyl and alkyl of up to 6 carbon atoms or bears the substituent 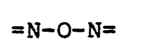 attached to the 2- and 3-positions (that is, to form a benzo-2,1,3-oxadiazole nucleus);
wherein Ar is phenylene, naphthylene, tetrahydronaphthylene, indanylene or pyridylene which is unsubstituted or which bears one or more substituents selected from halogeno, trifluoromethyl, hydroxy, amino, nitro, carbamoyl and cyano, and alkyl, alkenyl, alkoxy, alkenyloxy, alkoxyalkoxy, alkylthio, alkanoyl, carbamoylalkyl and alkanoylamino each of up to 6 carbon atoms;
wherein p is 0 or 1;
wherein q is 1, 2, 3 or 4;
wherein _{X} is -0-, -NH₋, -NHCO- or -CONH;
wherein X¹ is a direct link or is -O-, -S-, -NH- or -NHS⁰2^{-;}
and wherein Y is straight-or branched-chain alkylene of 1 to 12 carbon atoms which may optionally be interrupted by one or two groups selected from oxygen (-0-), sulphur (-S-), imino and substituted imino (-NR⁵ wherein R⁵ is hydrogen, alkyl or alkanoyl each of up to 10 carbon atoms, phenyl or aralkyl of up to 12 carbon atoms), phenylene, substituted phenylene, pyridylene, cycloalkylene of up to 6 carbon atoms, 1,4-piperazinediyl, 1,4-piperidinediyl and amido (-CONH- or -NHCO-) groups;
or an acid-addition salt thereof.

It will be observed that the dihydropyridine derivative of the invention possesses at least two asymmetric carbon atoms, namely the carbon atom of the -CHOH- group in the alkanolamine chain, and the carbon atom at the 4-position of the dihydropyridine nucleus, and it can therefore exist in racemic and optically-active forms. It is to be understood that this invention encompasses the racemic form of the dihydropyridine derivative and any optically-active form which possesses antihypertensive activity, it being a matter of common general knowledge how a racemic compound may be resolved into optically-active forms, and how the antihypertensive activity of these forms may be determined. It is further to be understood that beta-adrenergic blocking activity usually predominates in that optically-active form which has the "S" absolute configuration of the said -CHOH- group in the alkanolamine chain when p is 1 and the "R" absolute configuration when p is 0.

A suitable value for _{R}^{l}, _{R}², _{R}³, _{R}⁵ or a substituent in benzene ring A or Ar when it is alkyl is, for example, methyl, ethyl or isopropyl.

A suitable value for R^{l} or R² when it is alkoxyalkyl is, for example, methoxyethyl, ethoxyethyl or propoxyethyl.

A suitable value for R⁴ is, for example, isopropyl, s-butyl, t-butyl, 2-hydroxy-1,1-dimethylethyl, 2-hydroxy-1-methylethyl, 1-methyl-3-phenylpropyl, 1-methyl-2-phenoxyethyl or 2-isobutyramidoethyl;

A suitable halogeno substituent in the benzene ring A or in Ar is, for example fluoro, chloro or bromo.

A suitable value for the alkenyl, alkoxy, alkenyloxy, alkoxyalkoxy, alkylthio, carbamoylalkyl or alkanoylamino substituent in Ar is, for example, allyl, methoxy, ethoxy, isopropoxy, allyloxy, methoxyethoxy, methylthio, carbamoylmethyl or acetamido.

A suitable value for R⁵ when it is alkanoyl, or for an alkanoyl substituent in Ar is, for example,formyl, acetyl or benzoyl.

A suitable value for R⁵ when it is aralkyl, or for an aralkyl substituent in Ar is, for example, benzyl.

A suitable value for Y is, for example, straight-chain alkylene of the formula (-CH₂)ₙ-, wherein n is an integer from 1 to 12;
or -(CH₂)ₘC(CH₃)₂-;
or -(CH₂)ₘ-NH-(CH₂)ₙ-
-(CH₂)ₘ-N(CH₃)-(CH₂)ₙ-
-(CH₂)ₘ-O-(CH₂)ₙ- 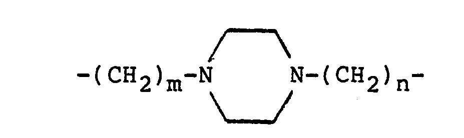 wherein m and n, which may be the same or different, each is 2,3,4 or 5; 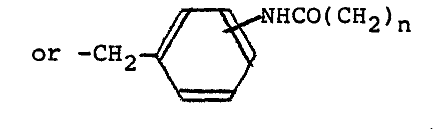
-(CH₂)ₘNHCO(C^{H}₂)ₙ- wherein m is 2, 3, 4 or 5 and n is 1, 2 or 3 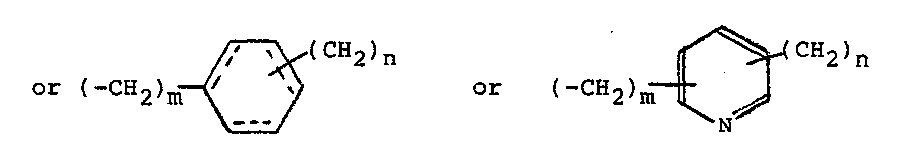 and wherein m and n, which may be the same or different, each is 1, 2, 3 or 4 and wherein the double bonds in the carbocyclic ring are optional (that is, cyclohexylene-or phenylene- bis-alkylene).

A preferred dihydropyridine derivative of the invention has the formula stated above wherein R¹ and _{R}², which may be the same or different, each is methyl or ethyl, wherein R³ is methyl, wherein R⁴ is isopropyl or t-butyl, wherein ring A is 3-nitrophenyl, wherein Ar is 1,4-phenylene, wherein p and q are both 1, wherein _{X} is -0-, wherein X^{l} is a direct link or -0- and wherein Y is alkylene of 2 to 4 carbon atoms which may be interrupted by -0- or -NHCO-, or is an acid-addition salt of such a compound. A particularly preferred compound is one wherein -Y-X¹⁻ is -(CH₂)₂NHCOCH₂-, -CH₂CH₂0- or -CH₂CH₂-0-CH₂CH₂-0-.

A suitable acid-addition salt of a dihydropyridine derivative of the invention is, for example, a salt derived from an inorganic acid, for example a hydrochloride, hydrobromide, phosphate or sulphate, or a salt derived from an organic acid, for example an oxalate, lactate, succinate, tartrate, acetate, salicylate, citrate, benzoate, beta-naphthoate or adipate.

Specific dihydropyridine derivatives of the invention are hereinafter described in the Examples. Of these, a preferred compound is ethyl 2-{2-[p-(2-hydroxy-3-isopropylaminopropoxy)phenylacetamido]-ethoxymethyl}-1,4-dihydro-5-methoxycarbonyl-6-methyl-4-m-nitrophenylpyridine-3-carboxylate.

The dihydropyridine derivatives of the invention may be manufactured by any chemical process known to be useful for the manufacture of chemically- analogous compounds.

One preferred process for the manufacture of a dihydropyridine derivative of the invention comprises the reaction of an epoxide of the formula:- 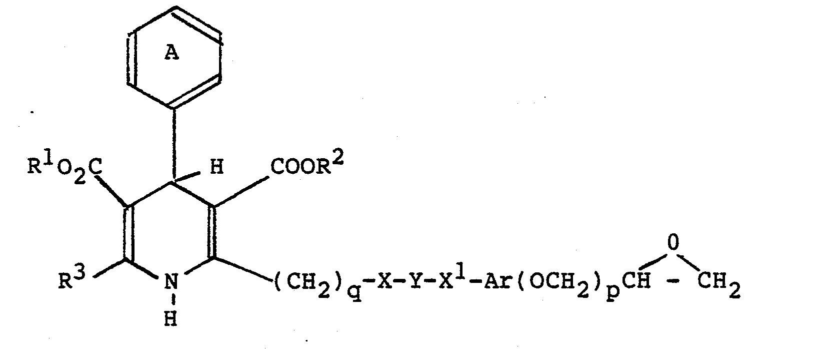 wherein A, _{R}l, _{R}2, _{R}³, _{X}, _{X}¹, _{Y}, _{A}r, p and q have the meanings stated above, with an amine of the formula:- 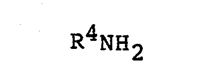 wherein R⁴ has the meaning stated above, or when p is 0, the reaction of said amine with a haloketone of the formula:- 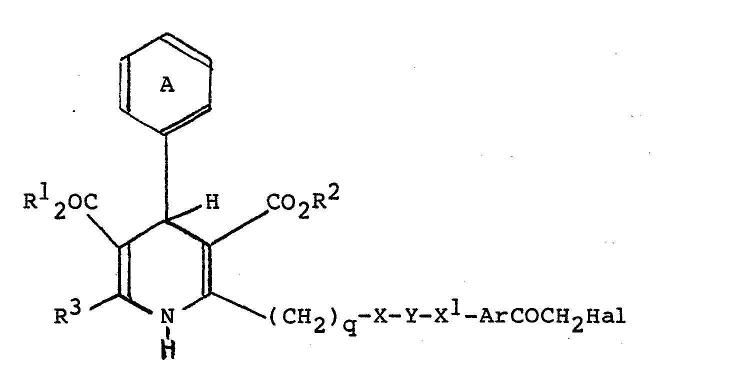 wherein A, _{R}1, _{R}², _{R}³, _{X}, _{X}¹, _{Y}, Ar and q have the meanings stated above and wherein Hal stands for a halogeno group, for example bromo, followed by reduction, for example with sodium borohydride, of the aminoketone thus obtained.

The reaction may be carried out in an alcoholic diluent or solvent, for example in isopropanol, at a temperature of up to the boiling point of said diluent or solvent.

A second preferred process for the manufacture of a dihydropyridine derivative of the invention wherein the group -Y- is alkylene interrupted as stated above comprises joining the two parts of the molecule at the point of interruption of Y. Thus, for example, when Y is alkylene interrupted by an amido group -NHCO-, the process comprises the reaction of an amine of the formula:- 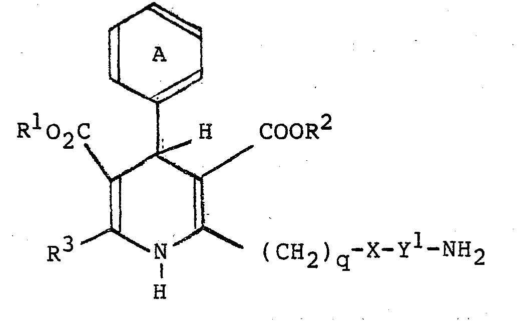 wherein A, _{R}^{l}, R², R³, q and _{X} have the meanings stated above, with an acid of the formula:- 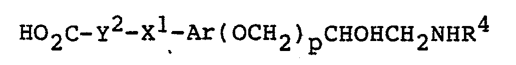 wherein _{X}¹, Ar, p and R⁴ have the meanings stated above, or with an activated derivative thereof and wherein _{Y}¹ and _{Y}² are such that -Y¹-NHCO-Y²- has the same meaning as stated above for Y.

A third process for the manufacture of a dihydropyridine derivative of the invention wherein X¹ is other than a direct link comprises the reaction of a compound of the formula:- 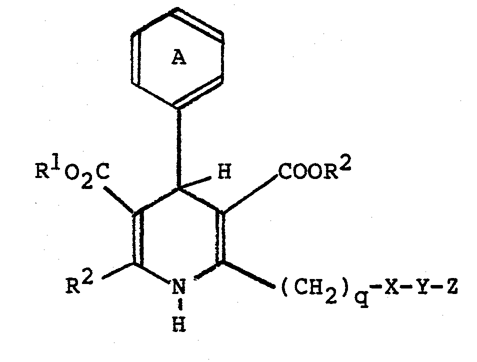 wherein A, R¹, R², R³, q, X and Y have the meanings stated above and wherein Z stands for a displaceable group, with a compound of the formula:- 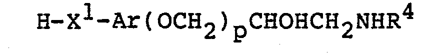 wherein _{R}⁴, _{X}¹, Ar and p have the meanings stated above.

A suitable value for Z is, for example, a halogeno group, for example a bromo or chloro group, or a sulphonyloxy group, for example a methanesulphonyloxy or p-toluenesulphonyloxy group.

A fourth process for the manufacture of a dihydropyridine derivative of the invention comprises the reaction of an aldehyde of the formula 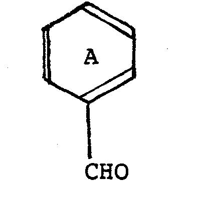 wherein A has the meaning stated above, an aminocrotonate of the formula 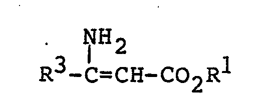 wherein _{R}^{l} and R³ have the meanings stated above and a ketoacid derivative of the formula 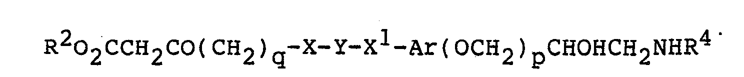 wherein _{A}r, p, q, R², R³, R⁴, X, X and Y have the meanings stated above.

This process may be carried out in a diluent or solvent at an elevated temperature, conditions conventionally used for the Hantszch synthesis of dihydropyridines.

As stated above, the dihydropyridine derivatives of the invention possess antihypertensive activity. This may be demonstrated by the ability of the compound to reduce the blood pressure of a spontaneously hypertensive rat, or of a rat made hypertensive by treatment with deoxycorticosterone acetate, or of a dog made hypertensive by the Goldblatt technique of unilateral nephrectomy and clipping of the contralateral kidney. These are all standard tests used to demonstrate antihypertensive effects of medicaments.

Some of the dihydropyridine derivatives of the invention possess beta-adrenergic blocking properties, some of them possess calcium ion slow-channel blocking properties and some of them possess both such activities. A preferred dihydropyridine derivative of the invention possesses both such activities. Beta-adrenergic blocking activity may be demonstrated in vivo by the ability of the compound to inhibit isoprenaline-induced tachycardia in a rat or cat, or in vitro by shifting to the right the dose- response curve of a guinea pig atrium to isoprenaline. Calcium ion slow channel blocking activity may be demonstrated in vitro by the ability of the compound to reduce spontaneous contraction in a rat portal vein preparation. These also are all standard tests used to demonstrate the stated activities.

Because of the beta-adrenergic blocking and/or calcium slow channel blocking properties a dihydropyridine of the invention may also be useful in the treatment of heart diseases such as angina pectoris and cardiac arrhythmias.

At. doses of a dihydropyridine derivative which produce effective antihypertensive activity in a rat or dog no symptom of toxicity is apparent.

The dihydropyridine derivative of the invention may be administered to warm-blooded animals, including man, in the form of a pharmaceutical composition comprising as active ingredient at least one dihydropyridine derivative of the invention, or an acid-addition salt thereof, in association with a pharmaceutically-acceptable diluent or carrier therefor.

A suitable composition is, for example, a tablet, capsule, aqueous or oily solution or suspension, dispersible powder, spray or aerosol formulation.

The pharmaceutical composition may contain, in addition to the dihydropyridine derivative of the invention, one or more drugs selected from sedatives, for example phenobarbitone, meprobamate, chloropromazine and the benzodiazepine sedative drugs, for example chlordiazepoxide and diazepam; vasodilators, for example glyceryl trinitrate, pentaerythritol tetranitrate, isosorbide dinitrate and hydralazine; diuretics, for example chlorthalidone, bendrofluazide, hydrochlorothiazide and chlorothiazide; other antihypertensive agents, for example reserpine, bethanidine and guanethidine; cardiac membrane stabilising agents, for example quinidine; agents used in the treatment of Parkinson's disease and other tremors, for example benzhexol; cardiotonic agents, for example digitalis preparations; and alpha-adrenergic blocking agents, for example phentolamine.

When used for the treatment of heart diseases, for example angina pectoris and cardiac arrhythmias, or for the treatment of hypertension in man, it is expected that the dihydropyridine derivative would be given to man at a total oral dose of between 20 mg. and 600 mg. daily, or at an intravenous dose of between 1 mg. and 20 mg.

Preferred oral dosage forms are tablets or capsules containing between 10 and 100 mg., and preferably 10 mg. or 50 mg., of active ingredient. Preferred intravenous dosage forms are sterile solutions of the dihydropyridine derivative or of a non- toxic acid-addition salt thereof, containing between 0.05% and 1% w/v of active ingredient, and more particularly containing 0.1% w/v of active ingredient.

The invention is illustrated but not limited by the following Examples:-Example 1

A mixture of ethyl 2-{2-[p-(2,3-epoxypropoxy)-phenylacetamido]ethoxymethyl}-1,4-dihydro-5-methoxycarbonyl-6-methyl-4-m-nitrophenylpyridine-3-carboxylate (0.8 g.), isopropylamine (5 ml.) and ethanol (10 ml.) was heated under reflux for 6 hours, cooled and evaporated to dryness under reduced pressure, and the residue was partitioned between ethyl acetate (10 ml.) and water (10 ml.). The organic layer was separated, dried over magnesium sulphate and evaporated to dryness under reduced pressure, and the residue was purified by flash chromatography on silica gel column (Merck 9385, 200 g.) using a 60:35:20:3 v/v/v/v mixture of toluene, ethanol, ethyl acetate and saturated aqueous ammonia solution as eluant. There was thus obtained as an oil ethyl 2-{2-[p-(2-hydroxy-3-isopropylaminopropoxy)phenyl- acetamido]ethoxymethyl}-1,4-dihydro-5-methoxycarbonyl-6-methyl-4-m-nitrophenylpyridine-3-carboxylate, the structure of which was confirmed by elemental analysis and proton magnetic resonance spectroscopy.

### Elemental analysis

Found: Cr60.8%; H,6.8%; N,8.2%. C₃₄H₄₄N₄0₁₀ requires: C,61.1%; H,6.6%; N,8.4%.

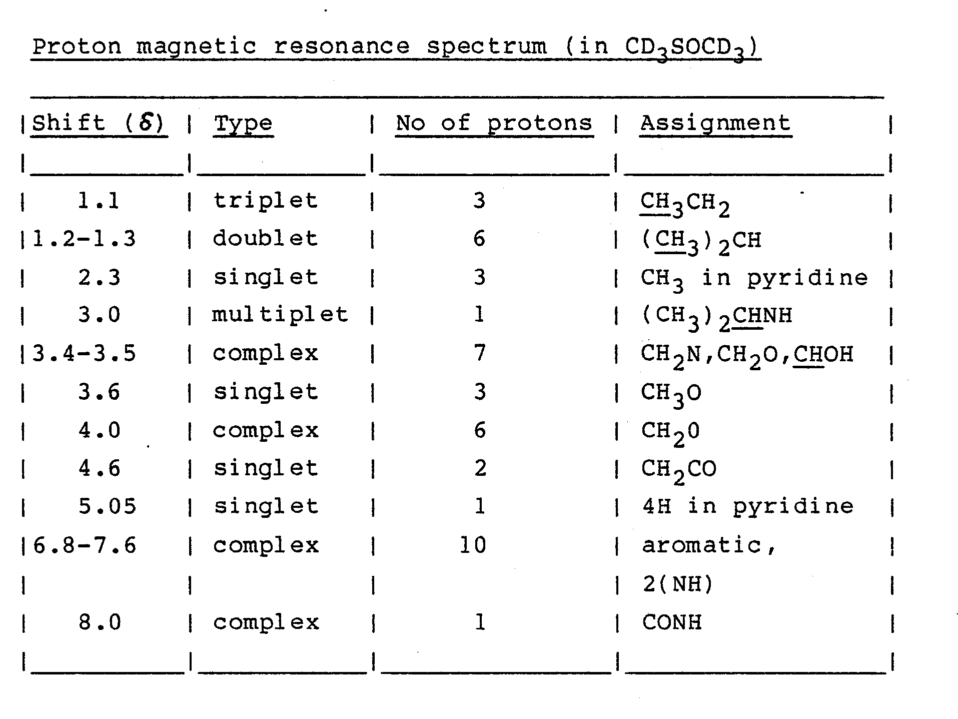

The ethyl pyridine-3-carboxylate used as starting material was obtained as follows:-A mixture of p-hydroxyphenylacetic acid (0.456 g.), dicyclohexylcarbodiimide (0.618 g.) and methylene chloride (20 ml.) was stirred at laboratory temperature for 10 minutes, 1-hydroxybenzotriazole (0.405 g.) was added and the mixture was stirred for a further 10 minutes. A solution in methylene chloride (15 ml.) of ethyl 2-(2-aminoethoxymethyl)-1,4-dihydro-5-methoxycarbonyl-6-methyl-4-m-nitrophenylpyridine-3-carboxylate (isolated from 1.6 g. of its maleate salt by shaking with 15 ml. of saturated aqueous sodium carbonate solution and then drying over magnesium sulphate) was added and the mixture was stirred at laboratory temperature for 20 hours and then filtered. The filtrate was washed with water, dried over magnesium sulphate and evaporated to dryness. The residue was stirred with ethyl acetate (10 ml.) and the mixture was kept at 0°C. for 20 hours and then filtered. The filtrate was evaporated to dryness and there was thus obtained as solid residue ethyl l,4-dihydro-2-[2-p-hydroxyphenylacetamido)ethoxymethyl]-5-methoxycarbonyl-6-methyl-4-m-nitrophenylpyridine-3-carboxylate.

A mixture of the above compound (1.2 g.), ethanol (20 ml.), epichlorohydrin (1.35 ml.) and aqueous N-sodium hydroxide solution (2.1 ml.) was stirred at laboratory temperature for 20 hours and then evaporated to dryness, and the residue was partitioned between ethyl acetate and water. The organic layer was separated, dried over magnesium sulphate and evaporated to dryness and there was thus obtained as residue the desired ethyl pyridine-3-carboxylate which was used without further purification.

### Example ₂.

The process described in Example 1 was repeated using t-butylamine in place of isopropylamine. There was thus obtained as an oil ethyl 2-{2-[p-(2-hydroxy-3-t-butylaminopropoxy)phenylacetamido]-ethoxymethyli-l,4-dihydro-5-methoxycarbonyl-6-methyl-4-m-nitrophenylpyridine-3-carboxylate, the structure of which was confirmed by elemental analysis and proton magnetic resonance spectroscopy.

### Example 3.

The process described in Example 1 was repeated using ethyl 2-{2-[2-p-(2,3-epoxypropoxy)-phenoxyethoxy]ethoxymethyl}-1,4-dihydro-5-methoxycarbonyl-6-methyl-4-m-nitrophenylpyridine-3-carboxylate and isopropylamine as starting materials. There was thus obtained as an oil ethyl 2-{2-[2-p-(2-hydroxy-3-isopropylaminopropoxy)phenoxyethoxy]ethoxymethyl}-1,4-dihydro-5-methoxycarbonyl-6-methyl-4-m-nitrophenyl- pyridine-3-carboxylate sesquihydrate, the structure of which was confirmed by elemental analysis, mass spectroscopy and proton magnetic resonance spectroscopy.

The ethyl pyridine-3-carboxylate used as starting material was prepared as follows:-A mixture of 3-oxapentamethylene-l,5-diol (10 g.), triphenylmethyl chloride (trityl chloride, 8.8 g.) and pyridine (100 ml.) was heated at 100°C. for 2 hours, diluted with water (100 ml.) and extracted with diethyl ether. The ethereal extract was washed successively with water, aqueous N-hydrochloric acid, water, 10% w/v aqueous sodium carbonate solution and water, dried and evaporated to dryness. The residue was crystallised from a mixture of toluene and petroleum ether (b.p. 60-80°C.) and there was thus obtained 2-(2-trityloxyethoxy)ethanol, m.p. l13-l14°C.

A solution of the above ethanol (6.3 g) in tetrahydrofuran (30 ml.) was added dropwise during 5 minutes to a stirred suspension of sodium hydride (1.9 g. of a 50% w/w suspension in paraffin oil from which the oil had been washed with petroleum ether) in tetrahydrofuran (10 ml.) and the mixture was stirred at laboratory temperature for 4.5 hours and then cooled to 0°C. Ethyl 4-chloro-3-oxobutyrate (2.98 g.) was added dropwise during 30 minutes and the mixture was allowed to warm up to laboratory temperature and was stirred for 18 hours. Ethanol (10 ml.) was added, the solution was poured into aqueous N-hydrochloric acid (30 ml.) and the mixture was extracted three times with diethyl ether (50 ml. each time). The combined ethereal extracts were washed with 10% w/v aqueous sodium carbonate solution and then with water, dried and evaporated to dryness.

A mixture of the ethyl 3-oxo-4-[2-(2-trityloxyethoxy)ethoxy]butyrate thus obtained as an oil (2.4 g.), methyl 3-aminocrotonate (0.58 g.), 3-nitrobenzaldehyde (0.76 g.) and ethanol (15 ml.) was heated under reflux for 18 hours and then evaporated to dryness, and the product was purified by flash chromatography on a silica gel (Merck 9385) column using a 4:1 v/v mixture of petroleum ether (b.p. 60-80°C.) and ethyl acetate as eluent. Aqueous 97% v/v acetic acid (10 ml.) was added and the mixture was heated at 100°C. for 30 minutes to remove the trityl group, and the mixture was evaporated to dryness.

Methanesulphonyl chloride (0.34 ml.) was added to a stirred mixture of the ethyl 1,4-dihydro-2-[2-(2-hydroxyethoxy)ethoxymethyl]-6-methyl-5-methoxycarbonyl-4-m-nitrophenylpyridine-3-carboxylate thus obtained (2.0 g.), triethylamine (0.45 g.) and methylene chloride (40 ml.) which was cooled to -4°C., and the mixture was stirred at 0°C. for 2 hours, washed with aqueous 10% w/v sodium carbonate solution and then with water, dried and evaporated to dryness. The residue (2.2 g.) was added to a mixture of quinol (1.34 g.), potassium hydroxide (0.27 g.) and dimethylformamide (10 ml.) which had been heated under reflux for 15 minutes. The mixture was heated under reflux for 2 hours, water (20 ml.) was added and the mixture was extracted with ethyl acetate (30 ml.). The extract was washed with aqueous 10% w/v sodium carbonate solution and then with water, dried and evaporated to dryness, and the residue was purified by flash chromatography on a silica gel (Merck 9385) column using a 1:1 v/v mixture of ethyl acetate and petroleum ether (b.p. 60-80°C.) as eluent.

A mixture of the residual ethyl 1,4-dihydro-2-[2-(2-p-hydroxyphenoxyethoxy)ethoxymethyl]-5-methoxycarbonyl-6-methyl-4-m-nitrophenylpyridine-3-carboxylate (0.75 g.), epichlorohydrin (0.8 ml.), aqueous N-sodium hydroxide solution (1.35 ml.) and methanol (7 ml.) was stirred at laboratory temperature for 80 hours, water (20 ml.) was added and the mixture extracted twice with ethyl acetate (20 ml. each time). The combined extracts were washed with water, dried and evaporated to dryness, and there was thus obtained the desired ethyl pyridine-3-carboxylate which was used without further purification.

### Example 4

The process described in Example 1 was repeated using ethyl 2-[2-p-(3-chloro-2-hydroxypropoxy)-phenoxyethoxymethyl]-1,4-dihydro-5-methoxycarbonyl-6-methyl-4-m-nitrophenylpyridine-3-carboxylate and isopropylamine as starting materials. There was thus obtained as an oil ethyl 2-[2-p-(2-hydroxy-3-isopropyl- aminopropoxy)phenoxyethoxymethyl]-l,4-dihydro-5-methoxycarbonyl-6-methyl-4-m-nitrophenylpyridine-3-carboxylate sesquihydrate, the structure of which was confirmed by elemental analysis, mass spectroscopy and proton magnetic resonance spectroscopy.

The ethyl pyridine-3-carboxylate used as starting material was obtained as follows:-A mixture of 2-p-hydroxyphenoxyethanol (4.0 g.), epichlorohydrin (18.2 ml.), aqueous N-sodium hydroxide solution (29 ml.) and ethanol (30 ml.) was stirred at laboratory temperature for 4 days and then evaporated to dryness, and the residue was shaken with water (30 ml.) and methylene chloride (30 ml.). The methylene chloride layer was passed through phase- separating paper and evaporated to dryness, and the residue was stirred with diethyl ether. The mixture was filtered and there was thus obtained as solid residue 2-p-(2,3-epoxypropoxy)phenoxyethanol, m.p. 60-62°C.

A solution of the above compound (4.0 g.) in tetrahydrofuran (10 ml.) was added during 30 minutes to a stirred suspension of sodium hydride (1.8 g. of a 50% w/w suspension in paraffin oil from which the paraffin had been washed with petroleum ether) in tetrahydrofuran ml.) and the mixture was stirred at laboratory temperature for 1 hour and then cooled to 0°C. A similar sequence of reactions to those described in Example 3 were then carried out using ethyl 4-chloro-3-oxobutyrate (15.34 g.) in the first reaction, and 3.8 g. of the ethyl 3-oxobutyrate thus obtained, methyl 3-aminocrotonate (1.17 g.), 3-nitrobenzaldehyde (1.53 g.) and methanol (20 ml.) in the second reaction. There was thus obtained the desired ethyl pyridine-3-carboxylate which was used without further purification.

## Claims

1. A dihydropyridine of the formula:- 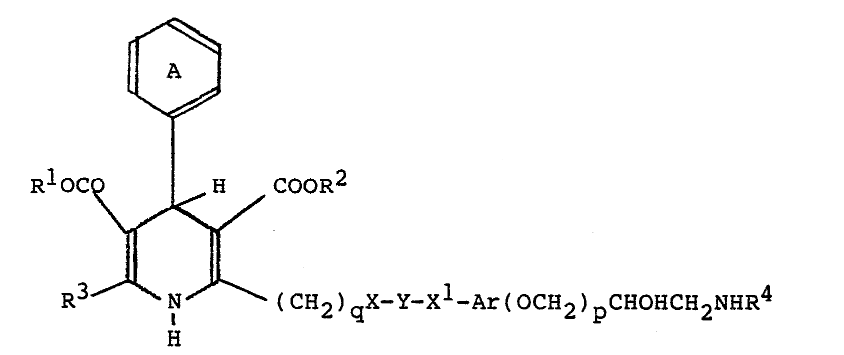 wherein R¹ and R², which may be the same or different, each is alkyl or alkoxyalkyl each of up to 6 carbon atoms:
wherein R³ is alkyl of up to 6 carbon atoms;
wherein R⁴ is alpha-branched-chain alkyl or hydroxyalkyl each of up to 6 carbon atoms, or alpha-branched chain arylalkyl or aryloxyalkyl each of up to 12 carbon atoms, or acylaminoalkyl wherein the acyl is is of up to 10 carbon atoms and the alkyl is of up to 6 carbon atoms; wherein benzene ring A bears one or more substituents selected from halogeno, cyano, nitro, trifluoromethyl and alkyl of up to 6 carbon atoms or bears the substituent 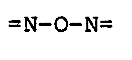 attached to the 2- and 3-positions (that is, to form a benzo-2,1,3-oxadiazole nucleus);
wherein Ar is phenylene, naphthylene, tetrahydronaphthylene, indanylene or pyridylene which is unsubstituted or which bears one or more substituents selected from halogeno, trifluoromethyl, hydroxy, amino, nitro, carbamoyl and cyano, and alkyl, alkenyl, alkoxy, alkenyloxy, alkoxyalkoxy, alkylthio, alkanoyl, carbamoylalkyl and alkanoylamino each of up to 6 carbon atoms;
wherein p is 0 or 1;
wherein q is 1, 2, 3 or 4;
wherein X is -O-, -NH-, -NHCO- or -CONH;
wherein X^{l} is a direct link or is -O-, -S-, -NH- or -NHS⁰2^{-;}
and wherein Y is straight-or branched-chain alkylene of 1 to 12 carbon atoms which may optionally be interrupted by one or two groups selected from oxygen (-0-), sulphur (-S-), imino and substituted imino (-NR^{S} wherein _{R}⁵ is hydrogen, alkyl or alkanoyl each of up to 10 carbon atoms, phenyl or aralkyl of up to 12 carbon atoms), phenylene, substituted phenylene, pyridylene, cycloalkylene of up to 6 carbon atoms, 1,4-piperazinediyl, 1,4-piperidinediyl and amido (-CONH- or -NHCO-) groups;
or an acid-addition salt thereof.

2. A dihydropridine as claimed in claim 1 wherein R¹ and _{R}², which may be the same or different, each is methyl or ethyl, wherein R³ is methyl, wherein R⁴ is isopropyl or t-butyl, wherein ring A is 3-nitrophenyl, wherein Ar is 1,4-phenylene, wherein p and q are both 1, wherein X is -0-, wherein X^{l} is a direct link or -0- and wherein Y is alkylene of 2 to 4 carbon atoms which may be interrupted by -0- or -NHCO-, or an acid-addition salt thereof.

3. A dihydropyridine as claimed in claim 2 wherein -Y-X¹- is -(CH₂)₂NHCOCH₂-, -CH₂CH₂0- or -CH₂CH₂-O-CH₂CH₂-O-.

4. The compound ethyl 2-{2-[p-(2-hydroxy-3-isoproplyaminopropoxy)phenylacetamido]ethoxymethyl}-1,4-dihydro-5-methoxycarbonyl-6-methyl-4-m-nitrophenyl- pyridine-3-carboxylate.

5. A process for the manufacture of a dihydropyridine, claimed in any of Claims 1 to 4, which comprises
(a) the reaction of an epoxide of the formula:- 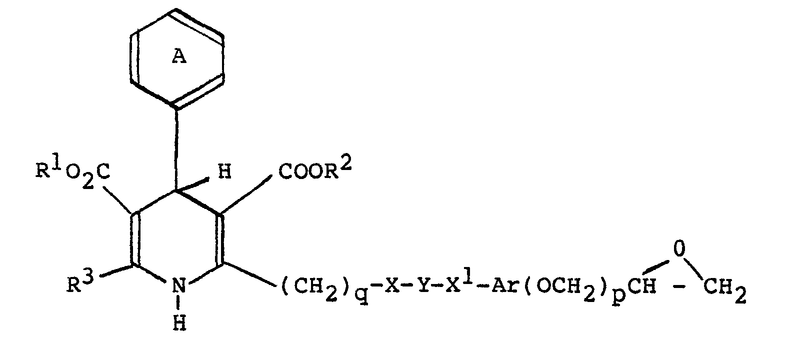 wherein A, R¹, R², R³, X, X¹, Y, Ar, p and q have the meanings stated in any of claims 1 to 3, with an amine of the formula:- 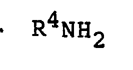 wherein R⁴ has the meaning stated in claim 1 or 2, or, when p is 0, the reaction of said amine with a haloketone of the formula:- 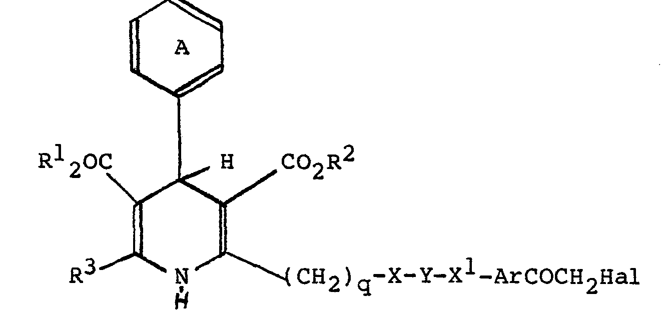 wherein A, R¹ , _{R}² , R³ , X, X¹ , Y, Ar and q have the meanings stated above and wherein Hal stands for a halogeno group, followed by reduction of the aminoketone thus obtained; or
(b) for the manufacture of a dihydropyridine wherein the group -Y- is alkylene interrupted by an amido group -NHCO-, the reaction of an amine of the formula:- 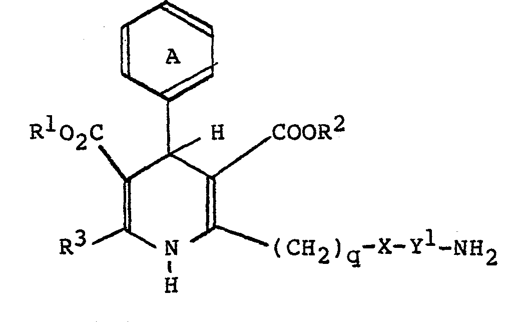 wherein A, _{R}^{l}, R², _{R}³, q and X have the meanings stated above, with an acid of the formula:- 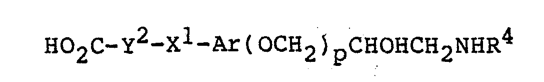 wherein X¹, _{A}r, p and R⁴ have the meanings stated above, or with an activated derivative thereof and wherein Y¹ and Y², are such that -Y¹⁻NHCO-Y²- has the same meaning as stated above for Y; or
(c) for the manufacture of a dihydropyridine wherein X¹ is other than a direct link the reaction of a compound of the formula:- 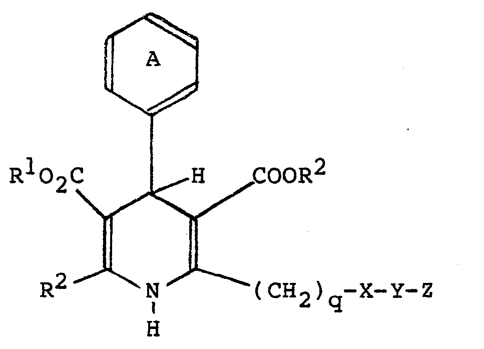 wherein A, R¹, R², R³, q, X and Y have the meanings stated above and wherein Z stands for a displaceable group, with a compound of the formula:- 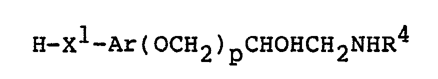 wherein R⁴, X¹, Ar and p have the meanings stated above; or
(d) the reaction of an aldehyde of the formula 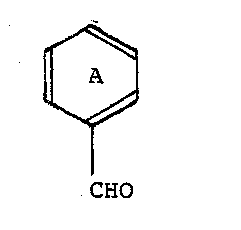 wherein A has the meaning stated above, an aminocrotonate of the formula 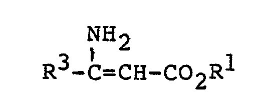 wherein _{R}¹ and R³ have the meanings stated above and a ketoacid derivative of the formula 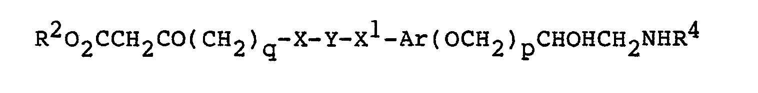 wherein _{Ar}, p, q, _{R}², _{R}³, _{R}⁴, X, X¹ and _{Y} have the meanings stated above.

6. A pharmaceutical composition comprising as active ingredient at least one dihydropyridine, claimed in any of claims 1 to 4, or an acid-addition salt thereof, in association with a pharmaceutically-acceptable diluent or carrier therefor.

7. A composition as claimed in claim 6 which is a tablet, capsule, aqueous or oily solution or suspension, dispersible powder, spray or aerosol formulation.

8. A composition as claimed in claim 6 or 7 which contains, in addition to the dihydropridine, one or more drugs selected from sedatives, vasodilators, diuretics, other antihypertensive agents, cardiac membrane stabilising agents, agents used in the treatment of Parkinson's disease and other tremors, cardiotonic agents, and alpha-adrenergic blocking agents.

9. The use of a compound, claimed in any of claims 1 to 4, for the manufacture of a medicament for producing an antihypertensive effect in a warm-blooded animal.

10. A method for the treatment of heart disease or hypertension in a warm-blooded animal which comprises administering to said animal an effective amount of a compound claimed in claim 1. /

1. A process for the manufacture of a dihydropyridine of the formula:- 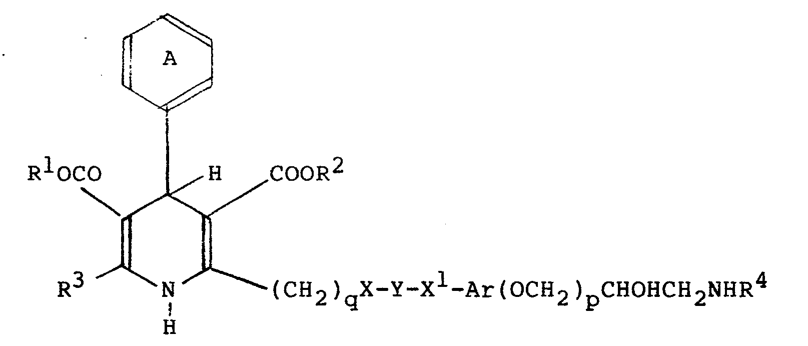 wherein R and R², which may be the same or different, each is alkyl or alkoxyalkyl each of up to 6 carbon atoms:
wherein R³ is alkyl of up to 6 carbon atoms;
wherein R⁴ is alpha-branched-chain alkyl or hydroxyalkyl each of up to 6 carbon atoms, or alpha-branched chain arylalkyl or aryloxyalkyl each of up to 12 carbon atoms, or acylaminoalkyl wherein the acyl is is of up to 10 carbon atoms and the alkyl is of up to 6 carbon atoms; wherein benzene ring A bears one or more substituents selected from halogeno, cyano, nitro, trifluoromethyl and alkyl of up to 6 carbon atoms or bears the substituent 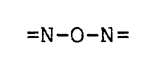 attached to the 2- and 3-positions (that is, to form a benzo-2,1,3-oxadiazole nucleus);
wherein Ar is phenylene, naphthylene, tetrahydronaphthylene, indanylene or pyridylene which is unsubstituted or which bears one or more substituents selected from halogeno, trifluoromethyl, hydroxy, amino, nitro, carbamoyl and cyano, and alkyl, alkenyl, alkoxy, alkenyloxy, alkoxyalkoxy, alkylthio, alkanoyl, carbamoylalkyl and alkanoylamino each of up to 6 carbon atoms;
wherein p is O or 1;
wherein q is 1, 2, 3 or 4;
wherein X is -O-, -NH-, -NHCO- or -CONH;
wherein X¹ is a direct link or is -O-, -S-, -NH- or -NHSO₂-;
and wherein Y is straight-or branched-chain alkylene of 1 to 12 carbon atoms which may optionally be interrupted by one or two groups selected from oxygen (-0-), sulphur (-S-), imino and substituted imino (-NR⁵ wherein R^{S} is hydrogen, alkyl or alkanoyl each of up to 10 carbon atoms, phenyl or aralkyl of up to 12 carbon atoms), phenylene, substituted phenylene, pyridylene, cycloalkylene of up to 6 carbon atoms, 1,4-piperazinediyl, 1,4-piperidinediyl and amido (-CONH- or -NHCO-) groups;
or an acid-addition salt thereof, characterised by:-(a) the reaction of an epoxide of the formula:- 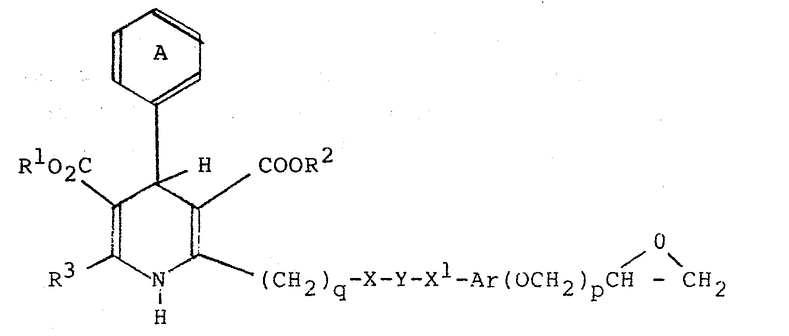 wherein A, R^{l}, R², R³, X, X^{l}, Y, Ar, p and q have the meanings stated above, with an amine of the formula:- 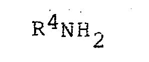 wherein R⁴ has the meaning stated above, or, when p is 0, the reaction of said amine with a haloketone of the formula:- 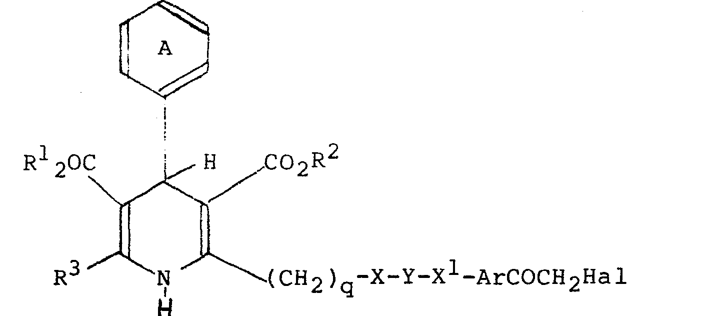 wherein A, R¹, R², R , X, X¹, Y, Ar and q have the meanings stated above and wherein Hal stands for a halogeno group, followed by reduction of the aminoketone thus obtained; or
(b) for the manufacture of a dihydropyridine wherein the group -Y- is alkylene interrupted by an amido group -NHCO-, the reaction of an amine of the formula:- 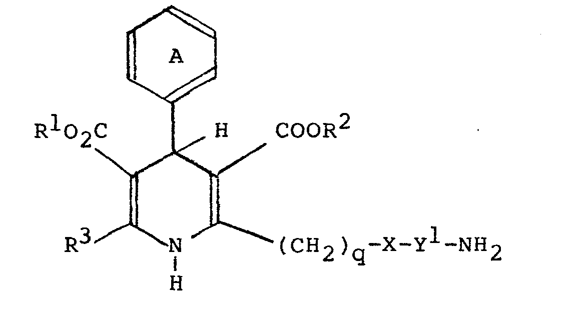 wherein A, R^{l}, R², R³, q and X have the meanings stated above, with an acid of the formula:- 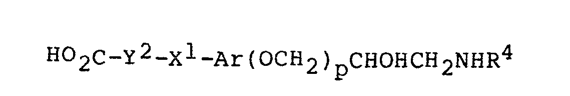 wherein X^{l}, Ar, p and R⁴ have the meanings stated above, or with an activated derivative thereof and wherein Y¹ and Y² are such that -Y¹⁻NHCO-Y²- has the same meaning as stated above for Y; or
(c) for the manufacture of a dihydropyridine wherein X¹ is other than a direct link the reaction of a, compound of the formula:- 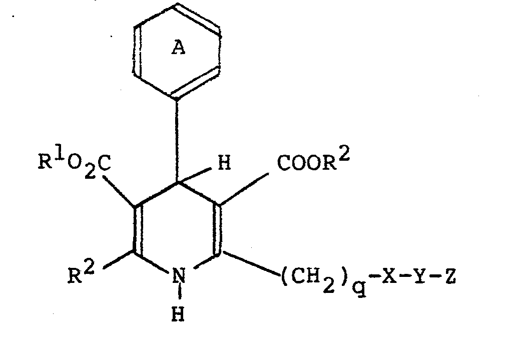 wherein A, R¹, R², R³, q, X and Y have the meanings stated above and wherein Z stands for a displaceable group, with a compound of the formula:- 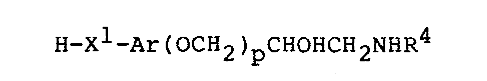 wherein R⁴, X¹, Ar and p have the meanings stated above; or
(d) the reaction of an aldehyde of the formula 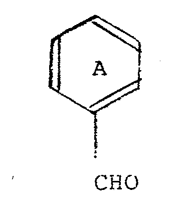 wherein A has the meaning stated above, an aminocrotonate of the formula 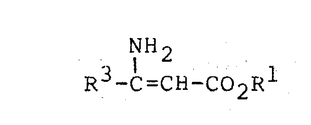 wherein R¹ and R³ have the meanings stated above and a ketoacid derivative of the formula 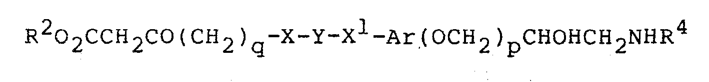 wherein Ar, p, q, R², R³, R⁴, X, X¹ and Y have the meanings stated above.

2. A process as claimed in claim 1 wherein in the starting materials R¹ and R², which may be the same or different, each is methyl or ethyl, R³ is methyl, R⁴ is isopropyl or t-butyl, ring A is 3-nitrophenyl, Ar is 1,4-phenylene, p and q are both 1, X is -0-, X¹ is a direct link or -0- and Y is alkylene of 2 to 4 carbon atoms which may be interrupted by -0- or -NHCO-.

3. A process as claimed in claim 2 wherein in the starting materials -Y-X¹- is - (CH₂)₂NHCOCH₂-, -CH₂CH₂0- or -CH₂CH₂-0-CH₂CH₂-0-.
